# EUROPEAN PATENT APPLICATION

(11) **EP 3 822 283 A1**
(43) Date of publication of application: **19.05.2021**
(21) Application number: 19383007.2
(22) Date of filing: 15.11.2019
(51) Int. Cl.: C07K 5/062, C07K 5/08, A61P 25/28, A61K 38/05, A61K 38/06

(54) **SEMAPHORIN 3A NEURODEGENERATION MODULATORS, COMPOSITIONS AND USES THEREOF**

(71) Applicant: Consejo Superior de Investigaciones Científicas (CSIC), 28006 Madrid (ES); Ramot at Tel-Aviv University Ltd., Tel-Aviv 6139201 (IL)
(72) Inventor: MESSEGUER PEYPOCH, Angel, 08034 Barcelona (ES); ALFONSO RODRIGUEZ, Ignacio, 08034 Barcelona (ES); BUJONS VILAS, Jordi, 08034 Barcelona (ES); PÉREZ RUIZ, Yolanda, 08034 Barcelona (ES); CORREDOR SÁNCHEZ, Miriam, 08034 Barcelona (ES); MOURE FERNANDEZ, Alejandra, 08034 Barcelona (ES); SOLOMON, Arieh, 6139201 Tel-Aviv (IL)
(74) Representative: Pons

(57) **Abstract**

Various neurodegenerative processes are entrained and maintained by signaling through Semaphorin 3A leading to apoptosis and loss of neuronal cells and tissues. Examples of such processes include glaucoma of the eye, stroke, ischemia of the optic nerve and other major nerves, retinal detachment and trauma. Small molecules in solution or in slow release systems are demonstrated to alleviate loss of retinal ganglial cells in models of CNS injury to the eye.

## Description

The present disclosure relates to anti-Semaphorin 3A compounds, as well as to their uses, especially as it relates to the modulation and/or treatment of neurodegeneration caused by any kind of insult.

### BACKGROUND ART

Neurodegeneration is a common cause of pathology in the nervous system especially in age. Typically, it is a process that initiates secondary to another physiological or environmental insult. The unusual aspect of neuro- versus other forms of tissue degeneration is that it often persists and extends even if the apparent cause of the insult is removed or reduced. This is, in part, because inflammatory processes are differently regulated in nervous tissues due to their immune privilege. An example is the bruising of the spinal cord in trauma leading to scarring that prevents nervous transmission. It would be expected that a similar injury to muscle, as opposed to nervous tissues, would have a different functional outcome.

Thus, the adult Central Nervous System (CNS) in higher vertebrates shows a limited capacity for anatomical and functional recovery after either acute or chronic neuropathies. Many studies suggest that the limited capacity for nerve regeneration is due to the presence of axonal growth inhibitory components in the CNS tissue. Several studies showed that CNS neurons possess growth potential supporting the hypothesis that inhibition of endogenous axonal growth inhibitors might allow regeneration of injured axons in adult CNS.

The central nervous system (CNS) encompasses a range of sensitive tissues and for the purpose of this disclosure we define it to include not only the brain but also the extensions to the major sensing organs including the eyes, ears, nose and spinal cord.

The chronic destructive stress responses of the CNS can be attributed to the fact that inflammation and edema in the form that is common in muscles and joints is extremely counterproductive in the CNS, which is, amongst others, highly pressure sensitive. Thus, the CNS immune response is characterized by isolation of the effected tissue and elimination of injured cells. In long lived organisms like humans, this response has the cost of significant loss of function. Accordingly, new methods and compositions that are useful for modulating the processes of neurodegeneration are of particular interest.

Degenerative processes of the CNS are complicated by a tendency to kill or remove injured tissue despite relief or reduction of the causative insult. A typical example is stroke where a transient reduction in blood supply evokes the loss of function in a wider region (the penumbra) despite the timely restoration of blood supply to the area. Other examples include spinal cord injury where even temporary vertebral misalignment evokes scarring and loss of axonal continuity and prevents re-connection of axons leading to disability. In glaucoma due to high intraocular pressure, the reduction of pressure and careful regulation thereof does not fully alleviate loss of retinal ganglial cells once this process is entrained. Thus, a key problem to solve in CNS degenerative disease is how to disengage the degenerative cascade once the causative insult has been resolved.

### DESCRIPTION OF INVENTION

The invention relates to compounds with capacity to modulate processes of neurodegeneration. The present disclosure includes compounds that inhibit signaling via the Sema3A pathway and pharmaceutical compositions containing these compounds or formulations containing varying concentrations of thereof.

The compounds reported here are useful in many respects. For example, they interrupt the signalling function of Sema3A and thus prevent loss of nervous tissue. They are suitable for use in the CNS in therapeutic mode to treat a condition that may lead to a degenerative process. Examples include insults to the auditory nerve, spinal cord, optic nerve and retina, as well as stroke. Unlike other therapies, these compounds fundamentally prevent a degenerative process from start. Thus, in cases of transient stress, the nervous tissue is maintained.

On the other hand, the compounds reported can be applied as solutions. They may also be provided as implants. They are adequately stable for pharmacological use when stored at the appropriate pH conditions.

In a first aspect, the invention refers to a compound according to Formula (I): 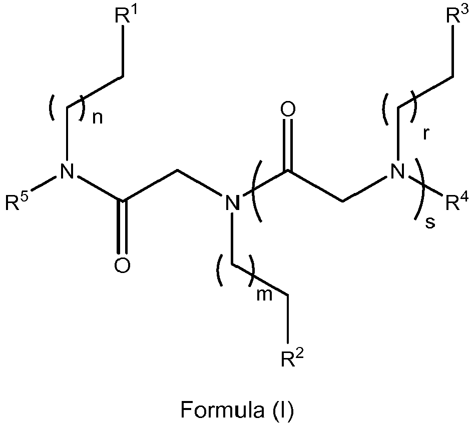 or a pharmaceutically acceptable salt, stereoisomer or solvate thereof, wherein:
R¹, R², and R³ are independently selected from optionally substituted aryl, optionally substituted heteroaryl, optionally substituted cyclyl, optionally substituted heterocyclyl, and -NR⁶R⁷;
R⁶ and R⁷ are each independently H, -(C₁-C₅)alkyl, -(C₁-C₃)alkyl-aryl, or -(C₁-C₃)alkyl-heteroaryl;
   or R⁶ and R⁷ are taken together with the nitrogen to which they are attached to form a 3- to 7-membered saturated or partially unsaturated monocyclic heterocycle, optionally containing one or two additional heteroatoms independently selected from O, S, N; wherein the heterocycle is optionally substituted with one or more substituents selected from halogen, -CF₃, hydroxyl, -NR⁸R⁹, -(C₁-C₃)alkyl, and -(C₁-C₃)alkoxy;
R⁴ is selected from -COOR¹⁰, -COR¹⁰, and -CONR⁸R⁹;
R⁵ is selected from H, -CH₂-COOR¹¹, and -CH₂-CONR⁸R⁹;
R⁸ and R⁹ are each independently H, -(C₁-C₅)alkyl or -(C₀-C₃)alkyl-aryl;
R¹⁰ is selected from -(C₁-C₅)alkyl, -(C₂-C₅)alkenyl, -(C₀-C₃)alkyl-aryl, -(C₀-C₃)alkyl-heteroaryl, -(C₀-C₃)alkyl-cyclyl, and -(C₀-C₃)alkyl-heterocyclyl;
R¹¹ is selected from H, -(C₁-C₅)alkyl, -(C₂-C₅)alkenyl, -(C₀-C₃)alkyl-aryl, -(C₀-C₃)alkyl-heteroaryl, -(C₀-C₃)alkyl-cyclyl, and -(C₀-C₃)alkyl-heterocyclyl;
n, m, and r are independently 0 to 3; and
s is 0 to 1.

In some embodiments of the compound of Formula (I), or a pharmaceutically acceptable salt, stereoisomer or solvate thereof, R¹, R², and R³ are independently selected from optionally substituted heterocyclyl and -NR⁶R⁷. In some embodiments, R¹, R², and R³ are independently selected from optionally substituted heterocyclyl and -NR⁶R⁷; and R⁶ and R⁷ are independently H or -(C₁-C₅)alkyl. In some embodiments, R¹, R², and R³ are independently 1-pyrrolidinyl, 1-methyl-pyrrolidin-2-yl, or diethylamino.

In some embodiments of the compound of Formula (I), or a pharmaceutically acceptable salt, stereoisomer or solvate thereof, R¹ is selected from optionally substituted heterocyclyl and -NR⁶R⁷. In some embodiments, R¹ is optionally substituted heterocyclyl. In some embodiments, R¹ is unsubstituted heterocyclyl. In some embodiments, R¹ is substituted heterocyclyl. In some embodiments, R¹ is heterocyclyl which is substituted with -(C₁-C₃)alkyl. In some embodiments, the R¹ is optionally substituted pyrrolidinyl. In some embodiments, R¹ is -NR⁶R⁷. In some embodiments, R¹ is -NR⁶R⁷; and R⁶ and R⁷ are independently H or -(C₁-C₅)alkyl. In some embodiments, R¹ is -NR⁶R⁷; and R⁶ and R⁷ are independently -(C₁-C₅)alkyl. In some embodiments, R¹ is 1-pyrrolidinyl, 1-methylpyrrolidin-2-yl, or diethylamino.

In some embodiments of the compound of Formula (I), or a pharmaceutically acceptable salt, stereoisomer or solvate thereof, R² is selected from optionally substituted heterocyclyl and -NR⁶R⁷. In some embodiments, R² is optionally substituted heterocyclyl. In some embodiments, R² is unsubstituted heterocyclyl. In some embodiments, R² is substituted heterocyclyl. In some embodiments, R² is heterocyclyl which is substituted with -(C₁-C₃)alkyl. In some embodiments, the R² is optionally substituted pyrrolidinyl. In some embodiments, R² is -NR⁶R⁷. In some embodiments, R² is -NR⁶R⁷; and R⁶ and R⁷ are independently H or -(C₁-C₅)alkyl. In some embodiments, R² is -NR⁶R⁷; and R⁶ and R⁷ are independently -(C₁-C₅)alkyl. In some embodiments, R² is 1-pyrrolidinyl, 1-methylpyrrolidin-2-yl, or diethylamino.

In some embodiments of the compound of Formula (I), or a pharmaceutically acceptable salt, stereoisomer or solvate thereof, R³ is selected from optionally substituted heterocyclyl and -NR⁶R⁷. In some embodiments, R³ is optionally substituted heterocyclyl. In some embodiments, R³ is unsubstituted heterocyclyl. In some embodiments, R³ is substituted heterocyclyl. In some embodiments, R³ is heterocyclyl which is substituted with -(C₁-C₃)alkyl. In some embodiments, the R³ is optionally substituted pyrrolidinyl. In some embodiments, R³ is -NR⁶R⁷. In some embodiments, R³ is -NR⁶R⁷; and R⁶ and R⁷ are independently H or -(C₁-C₅)alkyl. In some embodiments, R³ is -NR⁶R⁷; and R⁶ and R⁷ are independently -(C₁-C₅)alkyl. In some embodiments, R³ is 1-pyrrolidinyl, 1-methylpyrrolidin-2-yl, or diethylamino.

In some embodiments of the compound of Formula (I), or a pharmaceutically acceptable salt, stereoisomer or solvate thereof, R⁶ and R⁷ are taken together with the nitrogen to which they are attached to form a 3- to 7-membered saturated or partially unsaturated monocyclic heterocycle, optionally containing one or two additional heteroatoms independently selected from O, S and N. In some embodiments, the heterocycle is optionally substituted with one or more substituents selected from halogen, CF₃, hydroxyl, -NR⁸R⁹, (C₁-C₃)alkyl, and (C₁-C₃)alkoxy.

In some embodiments of the compound of Formula (I), or a pharmaceutically acceptable salt, stereoisomer or solvate thereof, n is 0 to 3. In some embodiments of the compound of Formula (I), or a pharmaceutically acceptable salt, stereoisomer or solvate thereof, n is 0 to 1, 0 to 2, 0 to 3, 1 to 2, 1 to 3, or 2 to 3. In some embodiments of the compound of Formula (I), or a pharmaceutically acceptable salt, stereoisomer or solvate thereof, n is 0, 1, 2, or 3.

In some embodiments of the compound of Formula (I), or a pharmaceutically acceptable salt, stereoisomer or solvate thereof, m is 0 to 3. In some embodiments of the compound of Formula (I), or a pharmaceutically acceptable salt, stereoisomer or solvate thereof, m is 0 to 1, 0 to 2, 0 to 3, 1 to 2, 1 to 3, or 2 to 3. In some embodiments of the compound of Formula (I), or a pharmaceutically acceptable salt, stereoisomer or solvate thereof, m is 0, 1, 2, or 3.

In some embodiments of the compound of Formula (I), or a pharmaceutically acceptable salt, stereoisomer or solvate thereof, r is 0 to 3. In some embodiments of the compound of Formula (I), or a pharmaceutically acceptable salt, stereoisomer or solvate thereof, r is 0 to 1, 0 to 2, 0 to 3, 1 to 2, 1 to 3, or 2 to 3. In some embodiments of the compound of Formula (I), or a pharmaceutically acceptable salt, stereoisomer or solvate thereof, r is 0, 1, 2, or 3.

In some embodiments of the compound of Formula (I), or a pharmaceutically acceptable salt, stereoisomer or solvate thereof, n, m, and r are each 1.

In some embodiments of the compound of Formula (I), or a pharmaceutically acceptable salt, stereoisomer or solvate thereof, s is 1. In some embodiments, s is 0.

In some embodiments of the compound of Formula (I), or a pharmaceutically acceptable salt, stereoisomer or solvate thereof, R⁴ is -COOR¹⁰. In some embodiments, R⁴ is - COR¹⁰. In some embodiments, R⁴ is -CONR⁸R⁹. In some embodiments, R⁴ is COOBn, wherein Bn represents a benzyl group.

In some embodiments of the compound of Formula (I), or a pharmaceutically acceptable salt, stereoisomer or solvate thereof, R⁵ is H. In some embodiments, R⁵ is -CH₂-COOR¹¹. In some embodiments, R⁵ is -CH₂-CONR⁸R⁹. In some embodiments, R⁵ is -CH₂-COOH. In some embodiments, R⁵ is -CH₂-CONH₂.

In some embodiments of the compound of Formula (I), or a pharmaceutically acceptable salt, stereoisomer or solvate thereof, R⁸ and R⁹ are independently H or -(C₁-C₅)alkyl. In some embodiments, R⁸ and R⁹ are each H. In some embodiments, R⁸ and R⁹ are each independently -(C₁-C₅)alkyl. In some embodiments, R⁸ is H; and R⁹ is -(C₁-C₅)alkyl.

In some embodiments of the compound of Formula (I), or a pharmaceutically acceptable salt, stereoisomer or solvate thereof, R¹⁰ is -(C₁-C₅)alkyl. In some embodiments, R¹⁰ is - (C₂-C₅)alkenyl. In some embodiments, R¹⁰ is -(C₀-C₃)alkyl-aryl. In some embodiments, R¹⁰ is -(C₀-C₃)alkyl-heteroaryl. In some embodiments, R¹⁰ is -(C₀-C₃)alkyl-cyclyl, R¹⁰ is - (C₀-C₃)alkyl-heterocyclyl. In some embodiments R¹⁰ is -(C₁-C₅)alkyl, or -(C₀-C₃)alkyl-aryl. In some embodiments, R¹⁰ is -CH₂-aryl. In some embodiments, R¹⁰ is -CH₂-phenyl.

In some embodiments of the compound of Formula (I), or a pharmaceutically acceptable salt, stereoisomer or solvate thereof, R¹¹ is H. In some embodiments, R¹¹ is -(C₁-C₅)alkyl. In some embodiments, R¹¹ is -(C₂-C₅)alkenyl. In some embodiments, R¹¹ is -(C₀-C₃)alkylaryl. In some embodiments, R¹¹ is -(C₀-C₃)alkyl-heteroaryl. In some embodiments, R¹¹ is -(C₀-C₃)alkyl-cyclyl, R¹¹ is -(C₀-C₃)alkyl-heterocyclyl. In some embodiments R¹¹ is H. In some embodiments R¹¹ is -(C₁-C₅)alkyl or -(C₀-C₃)alkyl-aryl. In some embodiments, R¹¹ is -(CH₂)-aryl. In some embodiments, R¹¹ is -CH₂-phenyl.

In some embodiments of the compound of Formula (I), or a pharmaceutically acceptable salt, stereoisomer or solvate thereof, is a compound selected from the group consisting of:
- 2-[2-(1-methylpyrrolidin-2-yl)ethyl]-5-(2-pyrrolidin-1-yl)ethyl-8-benzyloxycarbonyl-11,11-diethyl-3,6-dioxo-2,5,8,11-tetraazaundecancarboxamide;
- benzyl (2-(diethylamino)ethyl)(2-((2-((2-(1-methylpyrrolidin-2-yl)ethyl)amino)-2-oxoethyl)(2-(pyrrolidin-1-yl)ethyl)amino)-2-oxoethyl)carbamate;
- 2-(2-pyrrolidin-1-yl)ethyl-5-benzyloxycarbonyl-8,8-diethyl-3-oxo-2,5,8-triazaoctancarboxamide;
- 3-(2-pyrrolidin-1-yl)ethyl-6-benzyloxycarbonyl-9, 9-diethyl-4-oxo-3,6,9-triazanonanoic acid; and
- 3-(2-pyrrolidin-1-yl)ethyl-6-benzyloxycarbonyl-8-(1-pyrrolidyl)-4-oxo-3,6-diazaoctanoic acid.

In some embodiments of the compound of Formula (I), or a pharmaceutically acceptable salt, stereoisomer or solvate thereof, the pharmaceutically acceptable salt is the hydrochloride. In some embodiments, the pharmaceutically acceptable salt is the dihydrochloride.

In some embodiments of the compound of Formula (I), or a pharmaceutically acceptable salt, stereoisomer or solvate thereof, is a compound selected from the group consisting of:
- 3-(2-pyrrolidin-1-yl)ethyl-6-benzyloxycarbonyl-9, 9-diethyl-4-oxo-3,6,9-triazanonanoic acid dihydrochloride; and
- 3-(2-pyrrolidin-1-yl)ethyl-6-benzyloxycarbonyl-8-(1-pyrrolidyl)-4-oxo-3,6-diazaoctanoic acid dihydrochloride.

Compounds of Formula (I) can be prepared by following methods known by any person skilled in the field of organic synthesis, particularly through the general processes shown in the following reaction schemes and description thereof.

The starting materials are commercially available or the can be prepared by means of the methods of the literature. Unless indicated otherwise, the meaning of the groups R¹, R², R³ are those describe in the general Formula (I).

The general synthesis of compounds of Formula (I) is in Scheme 1.

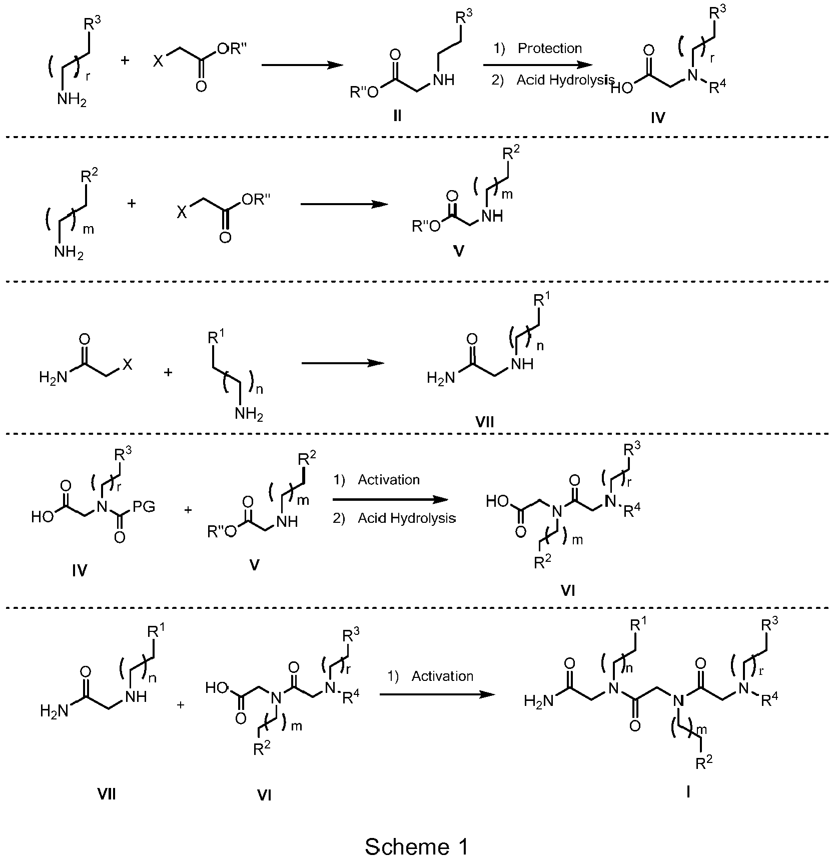

Scheme 1 describes a representative synthesis of the compounds described herein. First, the primary amine located at the final terminal amino fragment of the peptoid reacts with an α-haloacetic acid derivative in the presence of a base such as triethylamine or potassium carbonate to give the N-alkylated intermediate II. Intermediate II is subjected to amine protection followed by acid hydrolysis to afford intermediate IV. Intermediate V is synthesized in a similar manner to intermediate II. Alternatively, an α-haloacetamide is used to generate intermediate VII. Next, formation of the required amide between intermediates IV and V is carried out by using a suitable coupling agent, for example N,N'-dicyclohexylcarbodiimide (DCC), or N,N'-diisopropylcarbodiimide (DIC), using a tertiary amine as a base. Intermediate VI is rendered by acid hydrolysis. Finally, Intermediate VI is reacted with the amine fragment VII to obtain the corresponding compound of Formula (I).

Purification of intermediates and/or final compounds of Formula (I), if required, can be carried out by HPLC at semipreparative level.

In another aspect, the invention provides a pharmaceutical composition comprising: i) a compound of Formula (I) described herein or a pharmaceutically acceptable salt, stereoisomer or solvate thereof, and ii) a pharmaceutically acceptable excipient. The purpose of a pharmaceutical composition is to stabilize the active ingredient and to facilitate administration of a compound to an organism.

In an embodiment, one or more compounds of Formula (I) can be mixed with a pharmaceutically acceptable excipient to form a pharmaceutical composition for use in alleviating a disease or disorder, for example a disease or disorder that is associated with neurodegeneration.

In an embodiment, the pharmaceutical compositions to be used in the present methods can comprise one or more pharmaceutically acceptable excipients, preferably in the form of lyophilized formulations or water or saline aqueous solutions and aqueous dextrose and glycerol solutions, particularly for injectable solutions.

In an embodiment, the pharmaceutical compositions of the present invention may be specially formulated for administration in solid or liquid form, including those adapted for the following: (1) oral administration, for example, drenches (aqueous or non-aqueous solutions or suspensions), tablets, boluses, powders, granules, pastes, syrup, powder; (2) parenteral administration, for example, by subcutaneous, intramuscular or intravenous injection as, for example, a sterile solution or suspension; (3) topical application, for example, as a cream, ointment or spray applied to the skin; (4) intravaginally or intrarectally, for example, as a pessary, cream or foam; or (5) aerosol, for example, as an aqueous aerosol, liposomal preparation or solid particles containing the compound.

In another embodiment, the pharmaceutical composition is formulated in solution for injection and immediate release. In another embodiment, it is formulated for slow release. In another embodiment, it is formulated for delivery to a specific tissue such as the stroke penumbra, the inner ear or the vitreous of the eye.

In an embodiment, the pharmaceutical composition described herein comprises liposomes containing the compound, which can be prepared by any suitable method. Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter.

In another embodiment, the pharmaceutical composition described herein can be formulated in sustained-release format. Suitable examples of sustained-release preparations include semipermeable matrices of solid hydrophobic polymers containing the Compounds of the invention, which matrices are in the form of shaped articles, e.g. films, or microcapsules. Examples of sustained-release matrices include polyesters, hydrogels (for example, poly(2-hydroxyethyl-methacrylate), or poly(vinylalcohol)), copolymers of L-glutamic acid and 7 ethyl-L-glutamate, non-degradable ethylene-vinyl acetate, degradable lactic acid-glycolic acid copolymers such as the LUPRON DEPOT TM (injectable microspheres composed of lactic acid-glycolic acid copolymer and leuprolide acetate), sucrose acetate isobutyrate, and poly-D-(-)-3-hydroxybutyric acid.

In another embodiment, the pharmaceutical composition is delivered using a solid implant with a concentration of the Compound of the invention from about 0.1 to about 50% W/W. Implant refers to any object that is designed to be placed partially or wholly within a patient's body for one or more therapeutic or prophylactic purposes for delivering therapeutic agents. Implants are typically composed of biologically compatible synthetic materials (e.g., medical-grade stainless steel, titanium and other metals; polymers such as polyurethane, silicon, PCL (poly(e-caprolactone)), PLGA (poly (glycolic-co-lactic acid)), Polyglycol lactone (PGL), polylactide (PLA) and other materials).

In certain embodiments, these pharmaceutical compositions are suitable for local administration to a subject.

The pharmaceutical compositions to be used for in vivo administration must be sterile. This is readily accomplished by, for example, filtration through sterile filtration membranes. Therapeutic compositions are generally placed into a container having a sterile access port, for example, an intravenous solution bag or vial having a stopper pierceable by a hypodermic injection needle.

The pharmaceutical compositions described herein can be in unit dosage forms such as tablets, pills, capsules, powders, granules, solutions or suspensions, or suppositories, for oral, parenteral or rectal administration, or administration by inhalation or insufflation.

For preparing solid compositions such as tablets, the principal active ingredient can be mixed with a pharmaceutical carrier, e.g., conventional tableting ingredients such as corn starch, lactose, sucrose, sorbitol, talc, stearic acid, magnesium stearate, dicalcium phosphate or gums, and other pharmaceutical diluents, e.g., water, to form a solid pre-formulation composition containing a homogeneous mixture of a compound of the present disclosure, or a non-toxic pharmaceutically acceptable salt thereof. When referring to these pre-formulation compositions as homogeneous, it is meant that the active ingredient is dispersed evenly throughout the composition so that the composition may be readily subdivided into equally effective unit dosage forms such as tablets, pills and capsules. This solid pre-formulation composition is then subdivided into unit dosage forms of the type described above containing from 0.1 mg to about 500 mg of the active ingredient of the present disclosure. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer that serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

Suitable surface-active agents include, in particular, non-ionic agents, such as polyoxyethylenesorbitans (e.g., TweenTM 20, 40, 60, 80 or 85) and other sorbitans (e.g. SpanTM 20, 40, 60, 80 or 85). Compositions with a surface-active agent will conveniently comprise between 0.05 and 5% surface-active agent, and can be between about 0.1 and about 2.5% w/w. It will be appreciated that other ingredients may be added, for example mannitol or other pharmaceutically acceptable vehicles, if necessary.

Suitable emulsions may be prepared using commercially available fat emulsions, such as, for example, IntralipidTM, LiposynTM, InfonutrolTM, LipofundinTM and LipiphysanTM. The active ingredient may be either dissolved in a pre-mixed emulsion composition or alternatively it may be dissolved in an oil (e.g. soybean oil, safflower oil, cottonseed oil, sesame oil, corn oil or almond oil) and an emulsion formed upon mixing with a phospholipid (e.g. egg phospholipids, soybean phospholipids or soybean lecithin) and water. It will be appreciated that other ingredients may be added, for example glycerol or glucose, to adjust the tonicity of the emulsion. Suitable emulsions will typically contain up to 20% w/w. oil, for example, between about 5 and about 20%.

Pharmaceutical compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as set out above. In some embodiments, the compositions are administered by the oral or nasal respiratory route for local or systemic effect.

Compositions in preferably sterile pharmaceutically acceptable solvents may be nebulised by use of gases. Nebulised solutions may be breathed directly from the nebulising device or the nebulising device may be attached to a face mask, tent or intermittent positive pressure breathing machine. Solution, suspension or powder compositions may be administered, preferably orally or nasally, from devices which deliver the formulation in an appropriate manner. Any of the compositions provided herein may include any of the compounds of Formula (I) provided herein that inhibit signalling via the Sema3A pathway. In some embodiments, the compound is compound 2, 4, 8, 11, or 15.

In another aspect of the invention, the compounds of Formula (I) according to the present invention or a pharmaceutically acceptable salt, stereoisomer or solvate thereof may be used for treating or preventing a condition modulated by the inhibition of Sema3A signalling pathway.

Therefore, in another aspect, the present invention provides a compound of Formula (I) as defined above, or a pharmaceutically acceptable salt, stereoisomer or solvate thereof, for use as a medicament.

The invention also relates to the use of a compound of Formula (I) as defined above, or a pharmaceutically acceptable salt, stereoisomer or solvate thereof, for the manufacture of a medicament.

Some aspects of the disclosure provide a compound of Formula (I) as defined above, or a pharmaceutically acceptable salt, stereoisomer or solvate thereof that inhibits signalling via the Sema3A pathway for use in treating a subject in need thereof.

Some aspects of the disclosure provide the use of a compound of Formula (I) as defined above, or a pharmaceutically acceptable salt, stereoisomer or solvate thereof that inhibits signalling via the Sema3A pathway for treating a subject in need thereof.

Some aspects of the disclosure provide the use of a compound of Formula (I) as defined above, or a pharmaceutically acceptable salt, stereoisomer or solvate thereof that inhibits signalling via the Sema3A pathway in the formulation of a medicament for treating a subject in need thereof.

In some aspects, a subject to be treated with a compound of Formula (I) as defined above, or a pharmaceutically acceptable salt, stereoisomer or solvate thereof exhibits neurodegeneration. In some embodiments, a compound of Formula (I) as defined above, or a pharmaceutically acceptable salt, stereoisomer or solvate thereof is effective in treating a disease, disorder, or injury associated with neurodegeneration.

As used herein, the term "neurodegenerative disorder" refers generally to a neuron disease, disorder, or injury in which neurons (e.g., of the brain, spinal cord, and neurons of sensing organs such as the eyes, ears, and nose) do not function properly resulting in progressive loss, typically resulting in diminished or lost neuronal function. Neurodegenerative disorders include, without limitation, a trauma to the brain, eye, ear, or spinal cord. In some embodiments, the trauma is the result of an ischemia (e.g., an ischemic stroke) to the brain, eye, ear, or spinal cord. In some embodiments, the neurodegenerative disorder includes a chronic insult to the brain, eye, ear, or spinal cord. In some embodiments, the neurodegenerative disorder includes an acute insult to the brain, eye, ear, or spinal cord. In some embodiments, the neurodegenerative disorder includes overpressure, aggregative disease, or vascular disease. In some embodiments, the neurodegenerative disorder includes a thrombotic event, a vascular insufficiency, an inflammatory reaction to an infectious agent, a detachment of the retina, an injury to hair cells of the ear, a pressure to the spinal cord, a stroke, trauma to the brain or major sensory nerves, any infarct of a major nerve or system or an acute ischemia of a major nerve or system, Alzheimer's disease, Parkinson's disease, amyotrophic lateral sclerosis (ALS), multiple sclerosis, or dementia.

Some aspects of the disclosure include methods of treating a subject suffering from, or susceptible to, a disease, disorder, or injury associated with neurodegeneration, the method comprising administering to the subject an effective amount of a compound of the invention, a combination thereof, or a composition as described herein. In some embodiments, the subject has suffered a trauma that is the result of an ischemia (e.g., an ischemic stroke) to the brain, eye, ear, or spinal cord. In some embodiments, the subject has suffered a chronic insult to the brain, eye, ear, or spinal cord. In some embodiments, the subject has suffered an acute insult to the brain, eye, ear, or spinal cord. In some embodiments, the subject has suffered an injury as a result of overpressure, aggregative disease, or vascular disease. In some embodiments, the subject has suffered a thrombotic event, a vascular insufficiency, an inflammatory reaction to an infectious agent, a detachment of the retina, an injury to hair cells of the ear, a pressure to the spinal cord, a stroke, trauma to the brain or major sensory nerves, any infarct of a major nerve or system or an acute ischemia of a major nerve or system, Alzheimer's disease, Parkinson's disease, ALS, multiple sclerosis, dementia, or a combination thereof.

Another aspect of the present invention relates to a compound of Formula (I) for use in the treatment and/or prevention of a disorder or disease selected from the group consisting of a trauma of the brain, eye, ear, or spinal cord; an ischemia of the brain, eye, ear, or spinal cord; a chronic insult to the brain, eye, ear, or spinal cord such as overpressure, aggregative disease, vascular disease; an acute insult to the brain, eye, ear, or spinal cord such as thrombotic event, a vascular insufficiency; an inflammatory reaction to an infectious agent; a detachment of the retina, an injury to hair cells of the ear, a full or partial section or pressure to the spinal cord, a stroke, section of a major nerve or group of nerves.

In another aspect, the treatment results in a decrease in Sema3A signaling in the subject. In another embodiment, the treatment results in a decrease in or halting of neurodegeneration in the subject. In another embodiment, the effective amount is administered to the subject intravenously. In another embodiment, the effective amount is administered to the subject subcutaneously. In another embodiment, the effective amount is administered to a central nervous system of the subject. In another embodiment, the effective amount is administered to an eye of the subject. In another embodiment, the effective amount is administered to an ear of the subject. In another embodiment, the effective amount is administered to the subject on more than one occasion. In another embodiment, the effective amount is administered to the subject at least weekly. In another embodiment, the effective amount is administered to the subject at least monthly.

The present invention also relates to the use of a compound of Formula (I) as defined above, or a pharmaceutically acceptable salt, stereoisomer or solvate thereof, in the manufacture of a medicament for the treatment and/or prevention of a disorder or disease selected from the group consisting of a trauma of the brain, eye, ear, or spinal cord; an ischemia of the brain, eye, ear, or spinal cord; a chronic insult to the brain, eye, ear, or spinal cord such as overpressure, aggregative disease, vascular disease; an acute insult to the brain, eye, ear, or spinal cord such as thrombotic event, a vascular insufficiency; an inflammatory reaction to an infectious agent; a detachment of the retina, an injury to hair cells of the ear, a full or partial section or pressure to the spinal cord, a stroke, section of a major nerve or group of nerves.

In some embodiments the invention includes a method for inhibiting or preventing Sema3A protein from binding to a Sema3A receptor, the method comprising contacting the Sema3A with the compound described herein, or the pharmaceutical composition described herein. In an embodiment, the Sema3A receptor is selected from the group consisting of a neuropilin-1 (NRP-1), a NRP-2, a Plexin A1, a Plexin A2, a Plexin A3, and a combination thereof.

In another embodiment, the invention provides a method for reducing activity of a human Sema3A in a subject, the method comprising administering to the subject the compound described herein or the pharmaceutical composition described herein, in an amount that is effective for reducing Sema3A activity in the subject. In another embodiment, the subject is a human.

In another embodiment, the invention provides an in vitro method for reducing activation of a Sema3A receptor in a cell, the method comprising, delivering to the cell the compound described herein, or the pharmaceutical composition described herein, in an amount that is effective for reducing activation of a Sema3A receptor in the cell.

In another aspect, the invention relates to methods of treating a subject suffering from, or susceptible to, or preventing a disease, disorder, or injury associated with neurodegeneration, the method comprising administering to the subject an effective amount of a compound described herein, or a pharmaceutically acceptable salt, stereoisomer or solvate thereof, or composition described herein. In some embodiments, the subject has suffered a trauma that is the result of an ischemia (e.g., an ischemic stroke) to the brain, eye, ear, or spinal cord. In some embodiments, the subject has suffered a chronic insult to the brain, eye, ear, or spinal cord. In some embodiments, the subject has suffered an acute insult to the brain, eye, ear, or spinal cord. In some embodiments, the subject has suffered an injury as a result of overpressure, aggregative disease, or vascular disease. In some embodiments, the subject has suffered a thrombotic event, a vascular insufficiency, an inflammatory reaction to an infectious agent, a detachment of the retina, an injury to hair cells of the ear, a pressure to the spinal cord, a stroke, trauma to the brain or major sensory nerves, any infarct of a major nerve or system or an acute ischemia of a major nerve or system, Alzheimer's disease, Parkinson's disease, ALS, multiple sclerosis, dementia, or a combination thereof.

To practice any of the methods disclosed herein, an effective amount of the pharmaceutical composition described above can be administered to a subject (e.g., a human) in need of the treatment via a suitable route, such as intravenous administration, e.g., as a bolus or by continuous infusion over a period of time, by intramuscular, intraperitoneal, intracerebrospinal, subcutaneous, intra-articular, intrasynovial, intrathecal, oral, inhalation or topical routes. Commercially available nebulizers for liquid formulations, including jet nebulizers and ultrasonic nebulizers are useful for administration. Liquid formulations can be directly nebulized and lyophilized powder can be nebulized after reconstitution.

The subject to be treated by the methods described herein can be a mammal, more preferably a human. Mammals include, but are not limited to, farm animals, sport animals, pets, primates, horses, dogs, cats, mice and rats. A human subject who needs the treatment may be a human patient having, at risk for, or suspected of having a neurodegenerative disorder, such as those noted above. A subject having a neurodegenerative disorder can be identified by routine medical examination, e.g., laboratory tests, organ functional tests, CT scans, or ultrasounds. A subject suspected of having any of such disorder might show one or more symptoms of the disorder. A subject at risk for the disorder can be a subject having one or more of the risk factors for that disorder.

Empirical considerations, such as the half-life, generally will contribute to the determination of the dosage. Frequency of administration may be determined and adjusted over the course of therapy, and is generally, but not necessarily, based on treatment and/or suppression and/or amelioration and/or delay of a neurodegenerative disorder. Alternatively, sustained continuous release formulations of compounds of formula (I) may be appropriate. Various formulations and devices for achieving sustained release, for example those provided herein, would be apparent to the skilled artisan and are within the scope of this disclosure.

In one example, dosages for a compound of Formula (I) as described herein may be determined empirically in individuals who have been given one or more administration(s) of the compound. Individuals may be given incremental dosages of the compound of Formula (I).

Generally, for administration of any of the compounds of Formula (I) described herein, an initial candidate dosage can be about 2 mg/kg, however, due to organ specific administration, the dose based on body weight will be misleading. Typical dose ranges in the eye or brain will be in the order of from about 100 to about 50,000 µg, more preferably from about 500 to about 20,000 µg, or more preferably from about 1,000 to about 10,000 µg. For the purpose of the present disclosure, a typical daily dosage might range from about 0.1 µg/kg to about 3 µg/kg, from about 0.1 µg/kg to about 30 µg/kg, from about 0.1 µg/kg to about 300 µg/kg, from about 0.1 µg/kg to about 3 mg/kg, from about 0.1 µg/kg to about 30 mg/kg, or from about 0.1 µg/kg to about 100 mg/kg or more, depending on the factors mentioned above. For repeated administrations over several days or longer, depending on the condition, the treatment is sustained until a desired suppression of symptoms occurs or until sufficient therapeutic levels are achieved to alleviate a neurodegenerative disorder, or a symptom thereof.

In some embodiments, for an adult patient of normal weight, doses ranging from about 0.3 to about 5.00 mg/kg may be administered. The particular dosage regimen, e.g., dose, timing and repetition, will depend on the particular individual and that individual's medical history, as well as the properties of the individual agents.

Dosage amounts will typically be in the range of from about 0.0001 or 0.001 or 0.01 mg/kg/day to about 100 mg/kg/day, but can be higher or lower, depending upon, among other factors, the activity of the compound of the invention, its bioavailability, the mode of administration, and various factors discussed above. Dosage amount and interval can be adjusted individually to provide plasma levels of the compound of the invention, which are sufficient to maintain therapeutic or prophylactic effect. In cases of local administration or selective uptake, such as local topical administration, the effective local concentration of active compound cannot be related to plasma concentration. Skilled artisans will be able to optimize effective local dosages without undue experimentation.

The compound of the invention can be administered once per day, a few or several times per day, or even multiple times per day, depending upon, among other things, the indication being treated and the judgment of the prescribing physician.

For the purpose of the present disclosure, the appropriate dosage will depend on the compound employed, the type and severity of the neurodegenerative disorder, whether the compound is administered for preventive or therapeutic purposes, previous therapy, the patient's clinical history and response to the antagonist, and the discretion of the attending physician. In some embodiments, the administration of a compound of the invention may be essentially continuous over a preselected period of time or may be in a series of spaced dose, e.g., either before, during, or after developing a neurodegenerative disorder.

As used herein, the term "treating" refers to the application or administration of a composition including one or more active agents to a subject, who has a neurodegenerative disorder, a symptom of the neurodegenerative disorder, or a predisposition toward the neurodegenerative disorder, with the purpose to cure, heal, alleviate, relieve, alter, remedy, ameliorate, improve, or affect the neurodegenerative disorder, the symptom of the neurodegenerative disorder, or the predisposition toward the neurodegenerative disorder.

Alleviating a neurodegenerative disorder includes delaying the development or progression of the neurodegenerative disorder, or reducing severity of the neurodegenerative disorder. Alleviating the neurodegenerative disorder does not necessarily require curative results. As used therein, "delaying" the development of a neurodegenerative disorder means to defer, hinder, slow, retard, stabilize, and/or postpone progression of the neurodegenerative disorder. This delay can be of varying lengths of time, depending on the history of the neurodegenerative disorder and/or individuals being treated. A method that "delays" or alleviates the development of a neurodegenerative disorder, or delays the onset of the neurodegenerative disorder, is a method that reduces probability of developing one or more symptoms of the neurodegenerative disorder in a given time frame and/or reduces extent of the symptoms in a given time frame, when compared to not using the method. Such comparisons are typically based on clinical studies, using a number of subjects sufficient to give a statistically significant result.

"Development" or "progression" of a neurodegenerative disorder means initial manifestations and/or ensuing progression of the neurodegenerative disorder. Development of the neurodegenerative disorder can be detectable and assessed using standard clinical techniques. However, development also refers to progression that may be undetectable. For purpose of this disclosure, development or progression refers to the biological course of the symptoms. "Development" includes occurrence, recurrence, and onset. As used herein "onset" or "occurrence" of a disease/disorder associated with a neurodegenerative disorder includes initial onset and/or recurrence.

In another aspect, the present invention also provides kits for use in alleviating neurodegenerative disorders. Such kits can include one or more containers comprising a compound of Fomula (I), or compositions comprising a compound de Formula (I).

In some embodiments, the kit can comprise instructions for use in accordance with any of the methods described herein. The included instructions can comprise a description of administration of the compound of formula (I) to treat, delay the onset, or alleviate a target disease as those described herein. The kit may further comprise a description of selecting an individual suitable for treatment based on identifying whether that individual has a neurodegenerative disorder. In still other embodiments, the instructions comprise a description of administering a compound to an individual at risk of the neurodegenerative disorder.

The instructions relating to the use of the compound of formula (I) generally include information as to dosage, dosing schedule, and route of administration for the intended treatment. The containers may be unit doses, bulk packages (e.g., multi-dose packages) or sub-unit doses. Instructions supplied in the kits of the disclosure are typically written instructions on a label or package insert (e.g., a paper sheet included in the kit), but machine-readable instructions (e.g., instructions carried on a magnetic or optical storage disk) are also acceptable.

The label or package insert indicates that the composition is used for treating, delaying the onset and/or alleviating a neurodegenerative disorder. Instructions may be provided for practicing any of the methods described herein.

The kits of this disclosure are in suitable packaging. Suitable packaging includes, but is not limited to, vials, bottles, jars, flexible packaging (e.g., sealed Mylar or plastic bags), and the like. Also contemplated are packages for use in combination with a specific device, such as an inhaler, nasal administration device (e.g., an atomizer) or an infusion device such as a minipump. A kit may have a sterile access port (for example the container may be an intravenous solution bag or a vial having a stopper pierceable by a hypodermic injection needle). The container may also have a sterile access port (for example the container may be an intravenous solution bag or a vial having a stopper pierceable by a hypodermic injection needle). At least one active agent in the composition is a compound as those described herein.

Kits may optionally provide additional components such as buffers and interpretive information. Normally, the kit comprises a container and a label or package insert(s) on or associated with the container. In some embodiments, the disclosure provides articles of manufacture comprising contents of the kits described above.

The following specific embodiments are, therefore, to be construed as merely illustrative, and not limitative of the remainder of the disclosure in any way whatsoever.

As used herein, the terms "about" and "approximately," when used to modify a numeric value or numeric range, indicate that deviations of up to about 0.2%, about 0.5%, about 1%, about 2%, about 5%, about 7.5%, or about 10% (or any integer between about 1% and 10%) above or below the value or range (±) remain within the intended meaning of the recited value or range.

The terms "(C₁-C₃)alkyl" and "(C₁-C₅)alkyl" represent straight or branched saturated hydrocarbon chains containing from one to three or from one to five carbons. Examples of (C₁-C₅)alkyl include without limitation methyl, ethyl, propyl, isopropyl, butyl, isobutyl, sec butyl, tert butyl, 1-pentyl, 1-methylbutyl, 2-methylbutyl, 3-methylbutyl, 1,1-dimethylpropyl, 1,2-dimethylpropyl and 2,2-dimethylpropyl.

The term "(C₂-C₅)alkenyl" represents an unsaturated straight or branched hydrocarbon chain, containing from two to five carbon atoms and one or more double bonds, for example ethenyl, 1 propenyl, 2 propenyl, 1 butenyl, 2 butenyl, 3 butenyl and 1,3 butadienyl, 1 pentenyl, 2 pentenyl, 3 pentenyl, 4 pentenyl, 1-methyl-2-butenyl, 2-methyl-3-butenyl, 3-methyl-1-butenyl, 1,1-dimethyl-2-propenyl and 1,2-dimethyl-1-propenyl.

The term "aryl" or "Ar" refers to a hydrocarbon monocyclic, bicyclic or tricyclic aromatic ring system containing 6 to 14 carbons in the ring portion. Examples of such "aryl" include, but are not limited to, phenyl, naphthyl (including 1-naphthyl and 2-naphthyl), indenyl, anthracenyl, fenantryl, fluorenyl. The aryl may have optionally one or more substituents, preferably from one to four, selected independently from halogen, -CF₃, - OH, -NH₂, -NRR', -(C₁-C₃)alkyl, and -(C₁-C₃)alkoxy; where R and R' are independently - (C1-C3)alkyl, or R and R' are taken together with the nitrogen to which they are attached to form a heterocycle.

The term "heteroaryl" or "Hetₐᵣ" refers to a stable monocyclic, bicyclic or tricyclic aromatic ring system containing 5 to 14 members in the ring portion, that consists of carbon atoms and from one to four heteroatoms selected from the group consisting in N, O and S, preferably an aromatic ring of 5 or 6 members with one or more heteroatoms. Examples of such "heteroaryl" include, not limited to, thiazolyl, benzimidazolyl, benzothiazolyl, furanyl, isothiazolyl, indolyl, pyridyl, piperidinyl, piperazinyl, thiadiazolyl, pyrrolyl, pyrazolyl, imidazolyle, indazolyl, etc. The heteroaryl may be optionally substituted at any available position by one or more substituents selected from halogen, -CF₃, -(C₁-C₃)alkyl, and -(C₁-C₃)alkoxy.

The term "heterocyclyl" or "heterocycle" refers to a stable radical of a 4- to 14- membered ring system that consists of carbon atoms and from one to three heteroatoms selected from the group consisting in N, O and S, preferably a ring of 5- or 6- members with one or more heteroatoms. The heterocycle can be a system of monocyclic, bicyclic or tricyclic rings, which can include condensed ring systems, and the heterocyclyl radical can be saturated or partially unsaturated. The heterocyclyl may be optionally substituted at any available position by one or more substituents selected from halogen, -OH, -CF₃, -(C₁-C₃)alkyl, and -(C₁-C₃)alkoxy.

The term "cyclyl" or "carbocyclyl" refers to a stable radical of a 3- to 8- membered monocyclic or 7- to 14- membered bicyclic carbocyclic ring system, which may be saturated or partially unsaturated. Representative examples of a cyclyl group include cyclopropyl, cyclopentyl, cyclohexyl, cyclobutyl, cycloheptyl, cyclopentenyl, cyclopentadienyl, cyclohexenyl or cyclohexadienyl. The cyclyl may optionally substituted at any available position by one or more substituents selected from halogen, -OH, -CF₃, -(C₁-C₃)alkyl, and -(C₁-C₃)alkoxy.

"Alkoxy" refers to a radical bonded through an oxygen atom of the formula -O-alkyl, where alkyl is an alkyl chain as defined above.

"Halo" or "halogen" refers to bromo, chloro, fluoro or iodo. In some embodiments, halogen is fluoro or chloro. In some embodiments, halogen is fluoro.

"Haloalkyl" refers to an alkyl radical, as defined above, that is substituted by one or more halo radicals, as defined above, e.g., trifluoromethyl, difluoromethyl, fluoromethyl, trichloromethyl, 2,2,2-trifluoroethyl, 1,2-difluoroethyl, 3-bromo-2-fluoropropyl, 1,2-dibromoethyl, and the like.

The term "optional" or "optionally" means that the subsequently described event or circumstance may or may not occur, and that the description includes instances where said event or circumstance occurs and instances in which it does not. For example, "optionally substituted alkyl" means either "alkyl" or "substituted alkyl" as defined above. Further, an optionally substituted group may be un-substituted (e.g., -CH₂CH₃), fully substituted (e.g., -CF₂CF₃), mono-substituted (e.g., -CH₂CH₂F) or substituted at a level anywhere in-between fully substituted and mono-substituted (e.g., -CH₂CHF₂, -CH₂CF₃, -CF₂CH₃, -CFHCHF₂, etc.). It will be understood by those skilled in the art with respect to any group containing one or more substituents that such groups are not intended to introduce any substitution or substitution patterns (e.g., substituted alkyl includes optionally substituted cycloalkyl groups, which in turn are defined as including optionally substituted alkyl groups, potentially ad infinitum) that are sterically impractical and/or synthetically non-feasible. Thus, any substituents described should generally be understood as having a maximum molecular weight of about 1,000 g/mol, and more typically, up to about 500 g/mol.

Many compounds of this invention have one or more double bonds, or one or more asymmetric centers. Such compounds can occur as racemates, racemic mixtures, single enantiomers, individual diastereomers, diastereomeric mixtures, and cis- or trans- or E- or Z- double isomeric forms.

Further, the aforementioned compounds also include their N-oxides. The term "N-oxides" refers to one or more nitrogen atoms, when present in a compound, are in N-oxide form, i.e., N→O.

The phrase "pharmaceutically acceptable" refers to those compounds of the invention, compositions containing such compounds and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The term "pharmaceutically acceptable salts" is meant to include salts of the compounds disclosed herein which are prepared with relatively nontoxic acids or bases, depending on the particular substituents found on the compounds described herein. When compounds of the present invention contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds of the present invention contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydroiodic, or phosphorous acids and the like, as well as the salts derived from relatively nontoxic organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like. Certain specific compounds of the present invention contain both basic and acidic functionalities that allow the compounds to be converted into either base or acid addition salts. Other pharmaceutically acceptable excipients known to those of skill in the art are suitable for the present invention.

The neutral forms of the compounds disclosed herein may be regenerated by contacting the salt with a base or acid and isolating the parent compound in the conventional manner. The parent form of the compound differs from the various salt forms in certain physical properties, such as solubility in polar solvents, but otherwise the salts are equivalent to the parent form of the compound for the purposes of the present invention.

"Pharmaceutically acceptable solvate" refers to a composition of matter that is the solvent addition form. In some embodiments, solvates contain either stoichiometric or non-stoichiometric amounts of a solvent, and are formed during the process of making with pharmaceutically acceptable solvents such as water, ethanol, and the like. Hydrates are formed when the solvent is water, or alcoholates are formed when the solvent is alcohol. Solvates of compounds described herein are conveniently prepared or formed during the processes described herein. The compounds provided herein optionally exist in either unsolvated as well as solvated forms.

Combinations of substituents and variables envisioned by this invention are only those that result in the formation of stable compounds. The term "stable", as used herein, refers to compounds which possess stability sufficient to allow manufacture and which maintains the integrity of the compound for a sufficient period of time to be useful for the purposes detailed herein (e.g., treating a disease).

In another aspect the present invention relates to a mild and highly selective process for the *in situ* introduction of the O-, S- and N-nitrate group into compounds of Formula (I).

The term "pharmaceutically acceptable excipient" refers to a carrier, vehicle, diluent, buffer or adjuvant that is administered with the active ingredient composition. Such pharmaceutical excipients can be without limitation sterile liquids, such as water and oils, including those of petroleum, animal, vegetable, or synthetic origin, such as peanut oil, soybean oil, mineral oil, sesame oil, and similar; calcium carbonate calcium phosphate, various sugars and types of starch, cellulose derivatives, gelatin and polyethylene glycols; wetting agents and lubricants such as sodium lauryl sulfate, as well as coloring agents, flavouring agents, lubricants, excipients, tableting agents, stabilizers, antioxidants and preservatives, can also be present. Suitable pharmaceutical vehicles are described in "Remington's Pharmaceutical Sciences" by E.W. Martin, latest edition, which is incorporated herein by reference.

The term "active ingredient" refers to the compounds of the invention accountable for the biological effect.

Semaphorin 3 (also abbreviated "Sema3A") is a protein, which in humans is encoded by the SEMA3A gene (Gene ID: 10371). Sema3A is a member of the semaphorin family and encodes a protein with an Ig-like C2-type (immunoglobulin-like) domain, a PSI domain and a Sema domain. This secreted protein can function as either a chemorepulsive agent, inhibiting axonal outgrowth, or as a chemoattractive agent, stimulating the growth of apical dendrites. In both cases, the protein is vital for normal neuronal pattern development. Increased expression of this protein is associated with schizophrenia and is seen in a variety of human tumor cell lines. Also, aberrant release of this protein is associated with the progression of Alzheimer's disease. Typically, Sema3A is secreted by neurons and surrounding tissue to guide migrating cells and axons in the developing nervous system.

Human semaphorin 3A (Sema3A) (NCBI Reference Sequence: NP_006071.1):














 (SEQ ID NO: 1).

A Sema3A protein may also be a non-human Sema3A protein such as, for example, a murine Sema3A protein having a sequence as set forth in UniProtKB O08665; a canine Sema3A protein having a sequence as set forth in UniProtKB E2QX94, a bovine Sema3A protein having a sequence as set forth in UniProtKB F1MEW1, a rat Sema3A protein having a sequence as set forth in UniProtKB Q63548, a chicken Sema3A protein having a sequence as set forth in UniProtKB F1NVZ5, a pig Sema3A protein having a sequence as set forth in UniProtKB I3LPP7, or a horse Sema3A protein having a sequence as set forth in UniProtKB F6RIR4.

The term "agent" is used herein to denote a chemical compound, a mixture of chemical compounds, a biological macromolecule, or an extract made from biological materials. In another embodiment, the therapeutic or cytotoxic agents include, but are not limited to, pertussis toxin, taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof.

The term "regulate" and "modulate" are used interchangeably, and, as used herein, refers to a change or an alteration in the activity of a molecule of interest (e.g., the biological activity of Sema3A). Modulation may be an increase or a decrease in the magnitude of a certain activity or function of the molecule of interest. Exemplary activities and functions of a molecule include, but are not limited to, binding characteristics, enzymatic activity, cell receptor activation, and signal transduction.

Correspondingly, the term "modulator," as used herein, is a compound capable of changing or altering an activity or function of a molecule of interest (e.g., the biological activity of Sema3A). For example, a modulator may cause an increase or decrease in the magnitude of a certain activity or function of a molecule compared to the magnitude of the activity or function observed in the absence of the modulator. In certain embodiments, a modulator is an inhibitor, which decreases the magnitude of at least one activity or function of a molecule. Exemplary inhibitors include, but are not limited to small organic molecules.

The term "agonist", as used herein, refers to a modulator that, when contacted with a molecule of interest, causes an increase in the magnitude of a certain activity or function of the molecule compared to the magnitude of the activity or function observed in the absence of the agonist. Agonists of Sema3A may include any molecules, which bind to Sema3A and increase a function or activity of Sema3A.

The term "antagonist" or "inhibitor", as used herein, refers to a modulator that, when contacted with a molecule of interest causes a decrease in the magnitude of a certain activity or function of the molecule compared to the magnitude of the activity or function observed in the absence of the antagonist. Particular antagonists of interest include those that block or modulate the biological or immunological activity of Sema3A. Antagonists and inhibitors of Sema3A may include, but are not limited to molecules, which bind to Sema3A and decrease a function or activity of Sema3A.

The term "effective amount", as used herein, refers to the amount of each compound of Formula (I) required to confer therapeutic effect on the subject, either alone or in combination with one or more other active agents. Effective amounts vary, as recognized by those skilled in the art, depending on the particular condition being treated, the severity of the condition, the individual patient parameters including age, physical condition, size, gender and weight, the duration of the treatment, the nature of concurrent therapy (if any), the specific route of administration and like factors within the knowledge and expertise of the health practitioner.

Therapeutic effect is understood as to reduce or ameliorate the severity and/or duration of a disorder or one or more symptoms thereof, prevent the advancement of a disorder, cause regression of a disorder, prevent the recurrence, development, onset or progression of one or more symptoms associated with a disorder, detect a disorder, or enhance or improve the prophylactic or therapeutic effect(s) of another therapy (e.g., prophylactic or therapeutic agent)

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

All of the features disclosed in this specification may be combined in any combination. Thus, unless expressly stated otherwise, each feature disclosed is only a representative example of a generic series of equivalent or similar features.

### BRIEF DESCRIPTION OF THE DRAWINGS

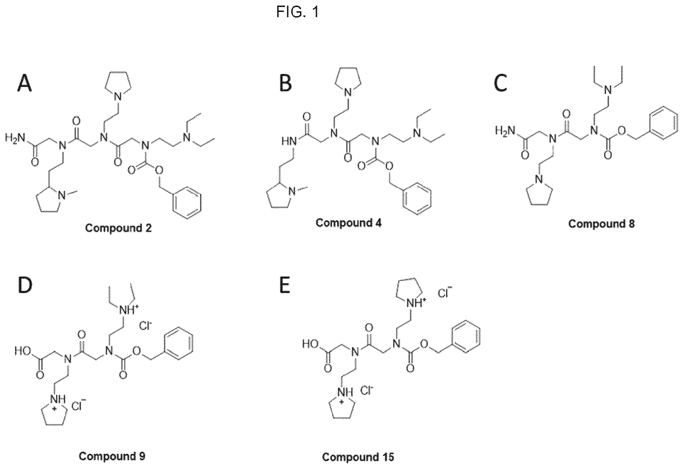

FIGS. 1A-E show structures of the Peptoid Sema 3A signaling inhibitors (Examples 1-3). FIG. 1A: Compound 2; FIG. 1B: Compound 4; FIG. 1C: Compound 8; FIG. 1D: Compound 9; and FIG. 1E: Compound 15.
FIG. 2 shows survival of rat RGCs following axotomy when treated with solutions of Compound 2 (0.025 µg/eye) compared to Sham or Vehicle controls. Statistical significance is shown. (See example 7).
FIG. 3 shows retina images of RGC survival. The control image (left) belong to retina of healthy rat. Vehicle and Compound 2 treated group were subjected to axotomy immediately before treatment. Data from 14 days after optic nerve surgery and 2 days after Di-Asp retrograde staining of RGC. Compound 2 intravitreal treatment presented 30% of RGC survival compared to healthy rats vs 1.5% RGC survival presented in the vehicle group. (see example 7).

### EXAMPLES

The application may be better understood by reference to the following non-limiting examples, which are provided as exemplary embodiments of the application. The following examples are presented in order to more fully illustrate embodiments and should in no way be construed, however, as limiting the broad scope of the application.

### EXAMPLE 1. Preparation of intermediates

### a) Preparation of the N-terminal monomer IV

### A1. N-benzyloxycarbonyl- N-[2-(diethylamino)ethyl]glycine hydrochloride (IVa)

First step: Synthesis of the N-terminal monomer is by alkylation of the commercial N,N-diethyl-1,2-etylenediamine. To a solution of N,N-diethylethylenediamine (3.6 mL, 25.8 mmol) and triethylamine (7.2 mL, 51.6 mmol) in 25 mL of THF, tert-butyl bromoacetate (3.8 mL, 25.8 mmol) was added at 0°C. Then the mixture was allowed to react overnight at room temperature. The solvent was evaporated to dryness and the residue obtained was treated with water and extracted with CH₂Cl₂. The organic layer was washed with water, dried and filtered. The desired product was purified by vacuum distillation to separate the mono- and di-alkylated products of the corresponding diamine to obtain tert-butyl N-[2-(diethylamino)ethyl]glycinate (IIa) (yield 49%).

Second step: Benzyl chloroformate (2.5 mL, 17.7 mmol) was added at 0°C to the solution of IIa (3.7 g, 16.1 mmol) and K₂CO₃ (6.7 g, 48.3 mmol) in 32 ml of dioxane: H2O (1:1). The mixture was allowed to react overnight at room temperature. The organic solvent was evaporated, and the residue was extracted with CH₂Cl₂ (3 x 10 mL). The joined organic fractions were dried and filtered. The solvent was removed under reduced pressure and the residue was purified by flash chromatography using DCM: MeOH as eluent to obtain tert-butyl N-benzyloxycarbonyl- N-[2-(diethylamino)ethyl]glycinate (IIIa) (87% yield).

Third step: IIIa (1.8 g, 4.9 mmol) was treated with 6.0 mL of HCl/dioxane (4 M, 24.0 mmol), and the mixture was allowed to react for 1 h at 60°C. Then, the solvent was removed under reduced pressure to give 1.6 g of N-benzyloxycarbonyl- N-[2-(diethylamino)ethyl]glycine hydrochloride (IVa) (99 % yield), which were used without further purification.

### A2. Synthesis of N-benzyloxycarbonyl- N-[2-(pyrrolidin-1-yl)ethyl]glycine hydrochloride (IVb)

Following an analogous procedure to that described for intermediate IVa, but using 1-(2-aminoethyl)pyrrolidine, intermediate N-benzyloxycarbonyl- N-[2-(pyrrolidin-1-yl)ethyl]glycine hydrochloride (IVb) was obtained.

### b) Preparation of central monomer V

### B1. Synthesis of tert-butyl N-[(2-pyrrolidin-1-yl)ethyl]glycinate (Va)

To a mixture of 1-(2-aminoethyl)pyrrolidine (3.0 mL, 23.7 mmol) and K₂CO₃ (9.8 g, 71 mmol) in 25 mL of THF, tert-butyl bromoacetate (3.5 mL, 23.7 mmol) was added at 0°C. The mixture was allowed to react for 30 min at room temperature, filtered and the solvent was evaporated to dryness. The residue obtained was treated with water (20 mL) and extracted with CH₂Cl₂ (25 mL), dried and filtered. The solvent was eliminated. The residue was distilled under vacuum to yield 2.9 g of tert-butyl N-[(2-pyrrolidin-1-yl)ethyl]glycinate (Va) (54%). HRMS (M + 1): calculated for C₁₂H₂₄N₂O₂: 229.1916. Found: 229.1913.

### c) Preparation of terminal monomer VII

### C1. Synthesis of 2-[2-(1-methylpyrrolidin-2-yl)ethylaminojacetamide (VIIa)

2 mL of 2-(2-Aminoethyl)-1-methylpyrrolidine (13.8 mmol) were dissolved in 75 mL of THF and 5.7 g of K₂CO₃ (41.4 mmol) were added. The mixture was cold at 0°C and bromoacetamide (1.9 g, 13.8 mmol) was added slowly. The new mixture was stirred for 2 h at room temperature, filtered and the THF was evaporated. The residue was distilled under vacuum to yield [2-(1-methylpyrrolidin-2-yl)ethylamino]acetamide (Vila). HRMS (M + 1): calcd. for C₉H₁₉N₃O: 186.1606. Found: 186.1592.

### C2. Synthesis of 2-((2-(pyrrolidin-1-yl)ethyl)amino)acetamide (VIIb)

Following an analogous procedure to that described for intermediate Vila but using 1-(2-aminoethyl)pyrrolidine, intermediate 2-((2-(pyrrolidin-1-yl)ethyl)amino)acetamide (VIIb) was obtained.

### d) Coupling of N-terminal and central monomers

### D1. Preparation of tert-butyl 2-(2-(((benzyloxy)carbonyl)(2-(diethylamino)ethyl)amino)-N-(2-(pyrrolidin-1-yl)ethyl)acetamido)acetate (VIa)

A solution of N-benzyloxycarbonyl-N-[2-(diethylamino)ethyl]glycine hydrochloride (IVa) (644 mg, 1.87 mmol) in 10 mL of anhydrous DCM, was treated with Et₃N (340 µL, 2.43 mmol) and *N*,*N*'-Dicyclohexylcarbodiimide (DCC) (503 mg, 2.43 mmol). The mixture was stirred 3 min at 60°C under microwave activation and tert-butyl *N*-[(2-pyrrolidin-1-yl)ethyl]glycinate (Va) (470 mg, 2.06 mmol) was added. The new mixture was stirred under microwave activation for 40 min at 60°C. The crude reaction mixture was filtered, the solid was washed with DCM, and the joined filtrate solution was evaporated in vacuum. The residue was purified by reverse-phase chromatography using CH₃CN/H₂O as eluent to give 504.2 mg of *tert*-butyl-2-(2-(((benzyloxy)carbonyl)(2-(diethylamino)ethyl)amino)-*N*-(2-(pyrrolidin-1*-yl)ethyl)acetamido)acetate* (Via) (52% yield). HRMS (M + 1): calculated for C₂₈H₄₆N₄O₅: 519.3546. Found: 519.3568.
1H NMR (400 MHz, rotamers, CDCl3 δ 7.35 - 7.25 (CH_{Ar}), 5.12-5,07 (CH₂ - Cbz), 4.27-3.88 (CH₂CO), 3.59 - 3.20 (4H, NCH₂), 2.76 - 2.38 (12H, CH₂), 1.85 - 1.66 (4H, CH₂ - pyrrolidine), 1.47-1.43 (9H, tert-butyl), 1.05-0.95 (3 xCH₃).

### D2. Preparation of tert-butyl 2-(2-(((benzyloxy)carbonyl)(2-(pyrrolidin-1-yl)ethyl)amino)-N-(2-(pyrrolidin-1-yl)ethyl)acetamido)acetate (Vlb)

Following an analogous procedure to that described for intermediate Via, but using intermediate IVb, *tert*-butyl-2-(2-(((benzyloxy)carbonyl)(2-(pyrrolidin-1-yl)ethyl)amino)-*N*-(2-(pyrrolidin-1-yl)ethyl)acetamido)acetate (Vlb) was obtained (29% yield). HRMS (M + 1): calculated for C₂₈H₄₄N₄O₅: 517.3390. Found: 517.3389.
¹H NMR (400 MHz, CDCl₃, rotamers) δ 7.36 - 7.27 (5H, CH_{Ar}), 5.14- 5.08 (2H, CH2-Cbz), 4.28 - 3.88 (4H, 2 x COCH₂), 3.57 - 3.24 (4 H, 2 x NCH₂), 2.79 - 2.39 (12H, NCH₂), 1.80 - 1.66 (8H, CH₂ - pyrrolidine), 1.45-1.42 (3 x CH₃).

### EXAMPLE 2. Preparation of Compound 9

*Tert*-butyl-2-(2-(((benzyloxy)carbonyl)(2-(diethylamino)ethyl)amino)-*N*-(2-(pyrrolidin-1-yl)ethyl)acetamido)acetate (Via) (0.50 g, 0.96 mmol) was treated with 2.2 mL of HCl/dioxane (4 M, 8.69 mmol), and the mixture was allowed to react for 1 h at 60°C. Then, the solvent was removed under reduced pressure. 515.4 g of 3-(2-pyrrolidin-1-yl)ethyl-6-benzyloxycarbonyl-9,9-diethyl-4-oxo-3,6,9-triazanonanoic acid dihydrochloride (Compound 9) (99 % yield) was obtained and it was used without further purification. HRMS (M + 1): calculated for C₂₄H₃₈N₄O₅: 463.2920. Found: 463.2908.
¹H NMR (400 MHz, rotamers, D₂O) δ 7.56 - 7.37 (CH_{Ar}), 5.21-5.10 (CH₂ - Cbz), 4.40-4.09 (NCH₂CO), 3.91 - 2.88 (16H, NCH₂), 2.16 - 1.91 (4H, CH₂ - pyrrolidine), 1.27-1.15 (6H, CH₃)

### EXAMPLE 3. Preparation of Compound 15

Following an analogous procedure to that described for Compound 9, but using intermediate VIb, 3-(2-pyrrolidin-1-yl)ethyl-6-benzyloxycarbonyl-8-(1-pyrrolidyl)-4-oxo-3,6-diazaoctanoic acid dihydrochloride (Compound 15) was obtained (99 % yield). HRMS (M + 1): C₂₈H₄₄N₄O₅: 461.2764. Found: 461.2756.
¹H NMR (400 MHz, D2O, rotamers,) δ 7.51 - 7.39 (5H, CH_{Ar}), 5.22 (2H, CH₂ - Cbz), 5.15-5.11 (2H, CH2 - Cbz), 4.38 - 4.10 (4H, COCH₂), 3.91 - 2.87 (16H, NCH₂), 2.16 - 1.81 (8H, CH₂ - pyrrolidine).

### EXAMPLE 4. Preparation of Compound 8

Following an analogous procedure to that described for intermediate Via, but using intermediate VIIb, 2-(2-pyrrolidin-1-yl)ethyl-5-benzyloxycarbonyl-8,8-diethyl-3-oxo-2,5,8-triazaoctancarboxamide (Compound 8) was obtained.

### EXAMPLE 5. Preparation of Compound 2

A solution of Compound 9 (290 mg, 0.54 mmol) in 4.0 mL of anhydrous DCM, was treated with Et₃N (230 µL, 1.63 mmol) and DCC (145.7 mg, 0.71 mmol). The mixture was stirred 2 min at 45°C under microwave activation and the [2-(1-methylpyrrolidin-2-yl)ethylamino]acetamide (Via) was added. The new mixture was stirred under microwave activation for 1.5 h at 45 °C. The crude reaction mixture was filtered, the solid was washed with DCM, and the joined filtrates were evaporated in vacuum. The residue was purified by reverse-phase chromatography using CH₃CN/H₂O as eluent (from 0% to 40% CH₃CN) to give 78.4 mg of 2-[2-(1-methylpyrrolidin-2-yl)ethyl]-5-(2-pyrrolidin-1-yl)ethyl-8-benzyloxycarbonyl-11,11-diethyl-3,6-dioxo-2,5,8,11-tetraazaundecancarboxamide (Compound 2) (23% yield). HRMS (M + 1): calculated for C₃₃H₅₅N₇O₅: 630.4343. Found: 630.4339.
¹H NMR (400 MHz, rotamers, CD₃CN) δ 7.40-7.26 (CH_{Ar}), 6.58-5.70 (NH), 5.10-5.04 (CH2 - Cbz), 4.35 - 3.83 (6H, COCH₂), 3.44 - 3.22 (6H, NCH₂), 2.95 (1H, CH₂NCH₃), 2.67 - 2.41 (12H, NCH₂), 2.24 - 2.21 (3H, NCH₃), 2.95 (m, 1H, CH₂NCH₃), 2.67 - 2.41 (m, 12H, NCH₂), 2.24 - 2.21 (3H, NCH₃), 2.08 (2H, 1H x CH + 1H x CH₂NCH₃), 1.93 - 1.52 (8H, 4H x CH₂ - pyrrolidine + 2H x CH₂CH₂NCH₃ + 2H x CH₂CH), 1.50 (m, 2H, CH₂CH), 0.99 - 0.89 (6H, CH₃).

### EXAMPLE 6. Preparation of Compound 4

Following an analogous procedure to that described for Compound 2 but using 2-(2-aminoethyl)-1-methylpyrrolidine instead of Vila, benzyl (2-(diethylamino)ethyl)(2-((2-((2-(1-methylpyrrolidin-2-yl)ethyl)amino)-2-oxoethyl)(2-(pyrrolidin-1-yl)ethyl)amino)-2-oxoethyl)carbamate (Compound 4) was obtained.

### EXAMPLE 7. Inhibition of Sema3A mediated RGC apoptosis in a rat optic nerve axotomy model

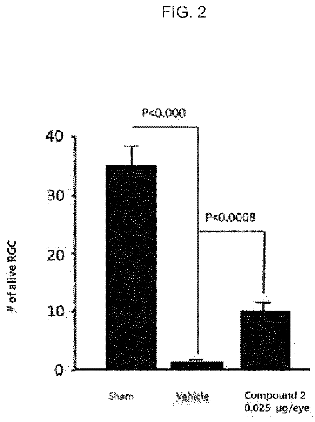

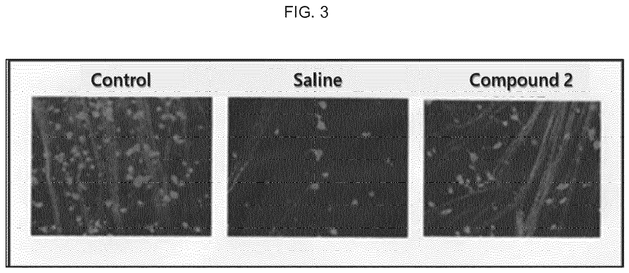

To evaluate the potential of the Compound 2 to inhibit Sema3a in a relevant small animal model, a rat axotomy study was carried out. The studies were carried out essentially as described in Shirvan et al., Id. Adult male Sprague-Dawley rats (12-15 weeks old, 300 g) were anesthetized (50 mg/kg xylazine and 35 mg/kg ketamine), and their left optic nerves were exposed by lateral canthotomy. Briefly, the conjuctivae were incised lateral to the cornea, and the retractor bulbus muscles were separated. Through a small opening in the meninges (50-100 µm), the nerve fibers were completely transected at a distance of 2-3 mm from the globe. The procedure was performed without damage to the nerve vasculature and optic nerve blood supply and with minimal damage to the meninges by the use of a specially designed glass dissector with a 200- µm tip and a smooth blunt edge (Solomon et al., 1996, Journal of Neuroscience Methods, 70, 21-5). The injury was unilateral in all animals, and the other eye served as a control.
The axotomized animals were divided into four groups of 4-6 rats each. Two groups were injected with Compound 2 at a single dose of 0.025 µg/eye in a volume of 4 µl PBS. The rats were injected immediately after surgery. The Sema3A inhibitor Compound 2 was injected into the vitreous body of the axotomized eye. Injection was performed using a glass pipette that was inserted into the eye globe. Insertion was at the corneal limbic border behind the lens, over the optic nerve head area and close to the retinal surface. Injection of the Sema3A inhibitor did not cause any signs of inflammation or irritation or any other changes that may indicate that this treatment is toxic. Clinical examination of the eyes was performed using a slit lamp, and the eyes were monitored for any signs of hyperemia, edema, dis- charge, fibrin, and other inflammatory parameters. At day 12 following axotomy of the optic nerve, the rats were anesthetized. Small crystals of the lipophilic neurotracer dye 4-(4-(didecylamino)styryl)-N-methylpyridinium iodide (4-Di- 10-Asp; Molecular Probes, Inc.) were dissolved (1 mM) in incomplete Freund's adjuvant (DIFCO®). This dye serves as a marker for living retinal ganglial cells (RGCs) because it is transmitted through the axonal network and stains the cell bodies of live neurons only, whereas nonviable neurons as well as other cell types such as endothelial cells remained unstained (Lazarov-Spiegler et al., 1999, Vision Research, 39, 169-75). The dye was applied to the transected nerve 0.5 mm from the proximal border of the transection site. The site of injury was visible by its grayish color in comparison with the rest of the nerve, which maintained its original color. Two days after dye application, retinas were excised, whole-mounted on Millipore filters, fixed in 4% paraformaldehyde in phosphate-buffered saline (PBS), and viewed under a fluorescent microscope using a fluorescein isothiocyanate filter. Two independent and blinded researchers counted the number of labeled RGC in flat-mounted retinas by fluorescent microscopy. For each retina, 20 representative microscopic fields were evaluated: 10 fields from the peripheral area and 10 fields from the central area (each field covering an area of 0.069 mm2). Central retinal areas were defined as located within two-thirds from the optic disc, and peripheral retinal areas were defined as located within one-third of the retinal radius. (These definitions were based on the apparent change in density of RGC between the two central and peripheral areas.) Retinas from eyes that were subjected to axotomy contained less RGC and were compared with intact retinas (FIG. 2, 3). Compound 2 was neuroprotective, since prevention of RGC loss was observed.

### ABBREVIATIONS

The following abbreviations were used as noted:
- MeOH:: methanol
- NaHCO₃:: sodium bicarbonate
- K₂CO₃:: potassium carbonate
- MS:: mass spectrometry
- DMSO:: dimethyl sulfoxide
- TLC:: thinlayer chromatography
- Et₃N:: triethylamine
- EtOAc:: ethyl acetate
- DCM:: dichloromethane
- NH₄Cl:: ammonium chloride
- THF:: tetrahydrofurane
- Na₂CO₃:: sodium carbonate
- EDCI:: N-Ethyl-N'-(3-dimethylaminopropyl)carbodiimide hydrochloride
- DMAP:: 4-dimethylamino pyridine
- PBS:: phosphate buffered saline

SEQUENCE LISTING

<110>  CSIC - Consejo Superior de Investigaciones Científicas.
       Ramot at Tel-Aviv University Ltd.

<120>  SEMAPHORIN 3A NEURODEGENERATION MODULATORS, COMPOSITIONS AND USES
        THEREOF

<130>  EP1641.1482

<160>  1

<170>  PatentIn version 3.5

<210>  1
<211>  771
<212>  PRT
<213>  Homo sapiens

<400>  1

## Claims

**1.** A Compound of Formula (I): 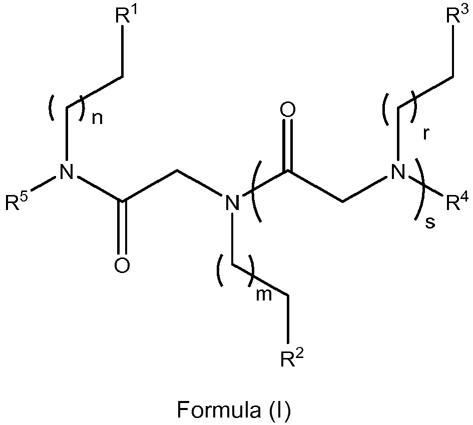 or a pharmaceutically acceptable salt, stereoisomer or solvate thereof, wherein:
R¹, R², and R³ are independently selected from optionally substituted aryl, optionally substituted heteroaryl, optionally substituted cyclyl, optionally substituted heterocyclyl, and -NR⁶R⁷;
R⁶ and R⁷ are each independently H, -(C₁-C₅)alkyl, -(C₁-C₃)alkyl-aryl, or -(C₁-C₃)alkyl-heteroaryl;
or R⁶ and R⁷ are taken together with the nitrogen to which they are attached to form a 3- to 7-membered saturated or partially unsaturated monocyclic heterocycle, optionally containing one or two additional heteroatoms independently selected from O, S, N; wherein the heterocycle is optionally substituted with one or more substituents selected from halogen, -CF₃, hydroxyl, -NR⁸R⁹, -(C₁-C₃)alkyl, and-(C₁-C₃)alkoxy;
R⁴ is selected from -COOR¹⁰, -COR¹⁰, and -CONR⁸R⁹;
R⁵ is selected from H, -CH₂-COOR¹¹, and -CH₂-CONR⁸R⁹;
R⁸ and R⁹ are each independently H, -(C₁-C₅)alkyl or -(C₀-C₃)alkyl-aryl;
R¹⁰ is selected from -(C₁-C₅)alkyl, -(C₂-C₅)alkenyl, -(C₀-C₃)alkyl-aryl, -(C₀-C₃)alkyl-heteroaryl, -(C₀-C₃)alkyl-cyclyl, and -(C₀-C₃)alkyl-heterocyclyl;
R¹¹ is selected from H, -(C₁-C₅)alkyl, -(C₂-C₅)alkenyl, -(C₀-C₃)alkyl-aryl, -(C₀-C₃)alkyl-heteroaryl, -(C₀-C₃)alkyl-cyclyl, and -(C₀-C₃)alkyl-heterocyclyl;
n, m, and r are independently 0 to 3; and
s is 0 to 1.

**2.** The compound according to claim 1, wherein R¹, R², and R³ are independently 1-pyrrolidinyl, 1-methyl-pyrrolidin-2-yl, or diethylamino.

**3.** The compound according to anyone of claims 1 or 2, wherein n, m, and r are 1.

**4.** The compound according to anyone of claims 1 to 3, wherein s is 1 or 0.

**5.** The compound according to anyone of claims 1 to 4, wherein R⁴ is -COOBn.

**6.** The compound according to anyone of claims 1 to 5, wherein R⁵ is H, -CH₂-CONH₂, or -CH₂-COOH, preferably R⁵ is -CH₂-CONH₂.

**7.** The compound according to claim 1, wherein the compound is selected from 2-[2-(1-methylpyrrolidin-2-yl)ethyl]-5-(2-pyrrolidin-1-yl)ethyl-8-benzyloxycarbonyl-11, 11-diethyl-3,6-dioxo-2,5,8,11-tetraazaundecancarboxamide,
benzyl (2-(diethylamino)ethyl)(2-((2-((2-(1-methylpyrrolidin-2-yl)ethyl)amino)-2-oxoethyl)(2-(pyrrolidin-1-yl)ethyl)amino)-2-oxoethyl)carbamate, 2-(2-pyrrolidin-1-yl)ethyl-5-benzyloxycarbonyl-8,8-diethyl-3-oxo-2,5,8-triazaoctancarboxamide, 3-(2-pyrrolidin-1-yl)ethyl-6-benzyloxycarbonyl-9,9-diethyl-4-oxo-3,6,9-triazanonanoic acid dihydrochloride, or 3-(2-pyrrolidin-1-yl)ethyl-6-benzyloxycarbonyl-8-(1-pyrrolidyl)-4-oxo-3,6-diazaoctanoic acid dihydrochloride.

**8.** A pharmaceutical composition comprising:
(a) a compound of Formula (I) as described in any of claims 1-7 and
(b) a pharmaceutically acceptable excipient.

**9.** A compound of Formula (I) as described in any of claims 1-7, or a pharmaceutically acceptable salt, stereoisomer or solvate thereof, for use as a medicament.

**10.** A compound of Formula (I) as described in any of claims 1-7, or a pharmaceutically acceptable salt, stereoisomer or solvate thereof, for use in the treatment and/or prevention of a disease, disorder, or injury associated with neurodegeneration.

**11.** A compound for use according to claim 10, wherein the disease, disorder, or injury is due to a chronic insult to a brain, an eye, an ear, a spinal cord, or a combination thereof, selected from the group consisting of overpressure, aggregative disease, vascular disease, and a combination thereof.

**12.** A compound for use according to claim 10, wherein the disease, disorder, or injury is due to acute insult to a brain, an eye, an ear, a spinal cord, or a combination thereof, selected from the group consisting of a thrombotic event, a vascular insufficiency, an inflammatory reaction to an infectious agent, a detachment of the retina, an injury to hair cells of the ear, a pressure to the spinal cord, a stroke, a trauma to the brain, a trauma to a sensory nerve, an infarct of a nerve, or an acute ischemia of a nerve.

**13.** A compound for use according to claim 10, wherein the neurodegeneration comprises Alzheimer's disease, Parkinson's disease, amyotrophic lateral sclerosis (ALS), multiple sclerosis, or dementia.

**14.** A compound for use according to any one of claims 9-13, **characterized in that** the compound is administered intravenously or subcutaneously.

**16.** A compound for use according to any one of claims 9-13, **characterized in that** the compound is administered on more than one occasion, at least weekly, or at least monthly.
